# FASCICULE DE BREVET EUROPEEN

(11) **EP 1 088 232 B1**
(45) Date de publication et mention de la délivrance du brevet: **14.09.2005**
(21) Numéro de dépôt: 99925112.7
(22) Date de dépôt: 16.06.1999
(51) Int. Cl.: G01N 33/574, G01N 33/573

(54) **PROCEDE ET TROUSSE DE DIAGNOSTIC PRECOCE DU CANCER**
VERFAHREN UND TESTSATZ ZUR FRÜHDIAGNOSE VON KREBS
METHOD AND KIT FOR EARLY DIAGNOSIS OF CANCER

(30) Priorité: 17.06.1998 FR 9807655
(43) Date de publication de la demande: 04.04.2001
(73) Titulaire: CENTRE NATIONAL DE LA RECHERCHE SCIENTIFIQUE (CNRS), 75794 Paris Cedex 16 (FR)
(72) Inventeur: BENISTANT, Christine, F-34750 Villeneuve lès Maguelonne (FR); ROCHE, Serge, F-34090 Montpellier (FR); CHAPUIS, Heliette, F-30127 Bellegarde (FR); BOURGAUX, Jean-François, F-34090 Montpellier (FR); MOTTET-AUSELO, Nicolas, F-30900 Nîmes (FR); BALI, Jean-Pierre, F-34980 Saint Gely du Fesc (FR)
(74) Mandataire: Breese, Pierre
(86) Numéro de dépôt international: PCT/FR1999/001444
(87) Numéro de publication internationale: WO 1999/066332

(56) Documents cités:
- WO-A-95/02187
- WO-A-95/24205
- S. ZRIHAN-LICHT ET AL.: "Association of Csk-homologous kinase (CHK) (formerly MATK) with HER-2/ErbB-2 in breast cancer cells" JOURNAL OF BIOLOGICAL CHEMISTRY., vol. 272, no. 3, 17 janvier 1997 (1997-01-17), pages 1856-1863, XP002066240 MD US cité dans la demande
- CHEMICAL ABSTRACTS, vol. 127, no. 18, 3 novembre 1997 (1997-11-03) Columbus, Ohio, US; abstract no. 244619, M. CHEDIN ET AL.: "Characterization of two different cytoplasmic protein tyrosine kinases from human breast cancer." page 252; colonne 1; XP002096755 & CARCINOGENESIS, vol. 18, no. 8, 1997, pages 1463-1472, cité dans la demande

## Description

La présente invention concerne le domaine du dépistage et du suivi du cancer. En effet, le problème majeur dans le traitement du cancer demeure son dépistage précoce qui permet la prise en charge thérapeutique dès le début de la maladie ce qui conduit dans de nombreux cas au succès du traitement.
De nombreux travaux ont montré que les cellules tumorales peuvent exprimer des substances susceptibles d'être détectées dans le sang et, en conséquence, pouvant être utilisées comme marqueurs tumoraux. Il est possible de distinguer deux grands types de telles substances :
- Les substances liées à la présence de tumeur mais qui ne sont pas impliquées dans sa formation. Il s'agit de protéines diverses (mucines, marqueurs CA 15-3 et CA 125), de fragments protéiques (fragments de cytokératine, marqueur CYFRA 21), d'enzymes (enolase neurospécifique, marqueur NSE), ou encore d'antigènes oncofoetaux comme les antigènes carcinoembryonnaires et les marqueurs ACE. Ces substances, présentes dans les cellules normales, sont altérées par l'activité tumorale et/ou ont accès à l'espace extracellulaire du fait de la nécrose tumorale. La détection de ces substances est déjà très utilisée en clinique, toutefois, elles n'apparaissent que lorsque la tumeur est bien en place et en conséquence elles ne peuvent pas être détectées précocement.
- Les substances directement liées à la formation des tumeurs. Parmi celles-ci, on peut citer tout particulièrement la protéine P53 qui est le produit d'un gène suppresseur de tumeur, et qui est altéré et/ou surexprimé dans les tumeurs. Il résulte de cette altération et/ou surexpression une modification du contrôle de la réparation de l'ADN et une plus grande susceptibilité des cellules à la transformation cellulaire (pour revue T. Soussi et al., 1994; *Int. J. Cancer:* 57, 1-9). Les altérations de la protéine P53 sont donc à l'origine de la formation de tumeurs dont elles constituent une étape précoce. En outre, la présence d'auto-anticorps dirigés contre la protéine P53 a été mise en évidence dans le sérum de patients atteints de cancer et la détection de ces auto-anticorps est en cours d'exploitation clinique (C. P. Wild et al., 1995, *Int. J. Cancer (Pred. Oncol.)*: 64, 176-181).

Les substances directement liées à la formation des tumeurs, comme la protéine P53 ou les auto-anticorps dirigés contre cette protéine constituent donc des marqueurs efficaces de cancers. Toutefois, dans la perspective d'une mise en oeuvre toujours plus rapide des traitements, il demeure utile d'identifier de nouveaux marqueurs plus précoces. En outre, les marqueurs déjà décrits, comme la protéine P53, sont caractéristiques de certains types de tumeurs et ne sont donc pas suffisamment polyvalents pour diagnostiquer tous les types de cancers.

La présente invention a précisément pour objet un nouveau marqueur des cancers plus précoce que la protéine P53 et permettant de détecter des cancers dont la protéine P53 ne constitue pas un marqueur.
Les travaux réalisés dans le cadre de la présente invention ont permis de mettre en évidence la présence dans le sérum de sujets atteints de cancer d'auto-anticorps dirigés contre une protéine cytoplasmique à activité enzymatique de type tyrosine kinase dénommée Csk pour "C-terminal Src Kinase". La protéine Csk est une protéine connue de longue date et dont le gène a été cloné et séquencé (S. Nada et al., 1991, *Nature* 351, 69-72 ; Bräuninger et al., *Proc. Natl. Acad. Sci.* USA, 1991, 88:10411-10415). La protéine Csk intervient dans la régulation négative de l'activité des enzymes tyrosine kinases de la famille Src (Okada et al., *J. Biol. Chem.,* 1991, 266:24249-24252; Nada et al., *Cell,* 1993, 73:1125-1135). Les enzymes de la famille Src sont des produits de proto-oncogènes cellulaires et sont impliqués dans la régulation de nombreuses fonctions biologiques et notamment la croissance cellulaire (Roche et al., *Science,* 1995, 269:1567-1569). Une surexpression ou une dérégulation de ces enzymes entraîne la formation de tumeurs chez l'animal. Chez l'homme, il a pu être montré que les enzymes de la famille Src sont activées et/ou surexprimées dans différents types de cancer : cancer du colon (Cartwright et al., *Proc. Natl. Acad. Sci.* USA, 1990, 87:558-562), mélanome (Loganzo et al., *Oncogene,* 1993, 8:2637-2644), cancer du sein (Lutrell et al., *Proc. Natl. Acad. Sci.* USA, 1994, 91:83-87; Chedin et al., *Carcinogenesis,* 1997, 18:1463-1472), cancer du colon et de l'ovaire (Budde et al., *Cancer Biochemistry and Biophysics,* 1994, 14:171-175), cancer de la vessie (Bénistant et al., en préparation). En outre, alors que Csk constitue un régulateur négatif des enzymes de la famille Src, il a été montré, paradoxalement, également une surexpression de Csk dans des tumeurs (Chedin et al., *Carcinogenesis,* 1997, 18:1463-1472).
Par analogie avec les travaux réalisés sur la protéine P53, les inventeurs ont maintenant recherché dans le sérum des sujets étudiés la présence éventuelle d'auto-anticorps dirigés contre les protéines Src et Csk. Cette étude était audacieuse car de nombreux autres produits de gènes sont connus pour s'accumuler dans les tumeurs (H-ras, ki-ras, myc, erb2, etc...) sans pour autant donner lieu à une génération d'auto-anticorps, et mis à part la protéine P53, il n'a jamais été montré la présence d'auto-anticorps contre des protéines impliqués dans la régulation cellulaire chez des sujets cancéreux. La mise en évidence de tels anticorps, sur laquelle est fondée la présente invention constitue donc une véritable découverte. Par ailleurs, le fait d'une part, qu'il existe des auto-anticorps anti-Csk chez certains patients, et d'autre part, que chez ces mêmes patients l'enzyme Src soit activée, suggère très fortement que le gène CSK pourrait être muté dans les tumeurs. Ainsi, la protéine Csk mutée constituerait un élément inconnu à l'organisme entraînant la mobilisation du système immunitaire comme cela semble être le cas pour la protéine P53. En outre, la protéine Csk mutée ne jouerait plus son rôle de régulateur négatif de enzymes de la famille Src qui deviendrait alors oncogénique. De plus, il n'est pas impossible que Csk ait d'autres fonctions dans la cellule, fonctions qui pourraient être altérées par une mutation de Csk, ou bien que Csk mutée agisse sur d'autres substrats que les enzymes de la famille Src perturbant alors diverses fonctions biologiques conduisant à une transformation cellulaire.

La présente invention a donc pour objet un procédé de diagnostic précoce d'un cancer dans lequel la protéine Src est activée chez un patient consistant à identifier par toute méthode appropriée la présence d'auto-anticorps dirigés contre une protéine Csk dans un échantillon biologique prélevé chez ledit patient.
On entend par protéine Csk la protéine Csk décrite par S. Nada et al., 1991 (*Nature* 351, 69-72 ; Bräuninger et al., *Proc. Natl. Acad, Sci*. USA, 1991, 88:10411-10415). D'autres enzymes appartiennent à la famille de Csk, comme celle désignée selon les auteurs Ctk, Ntk, Chk, Matk (Klages et al., Proc. *Natl. Acad. Sci.* USA, 1994, 91:2597-2601; Chow et al., *Proc. Natl. Acad. Sci.* USA, 194, 4975-4979; Davidson et al., *J. Biol. Chem*., 1997, 272:1856-1863), désignée ci-après par commodité Ctk. La protéine Ctk est produite par un autre gène que celui de la Csk.

Une méthode tout particulièrement appropriée pour identifier la présence d'auto-anticorps anti-Csk selon l'invention est fondée sur une réaction immunologique entre lesdits auto-anticorps et les protéines Csk, ou une forme modifiée, fragment(s) ou conjugués de ceux-ci, comme par exemple la technique ELISA. Celle-ci consiste à adsorber au fond de puits de plaques ELISA la protéine Csk et une protéine de référence (la BSA par exemple), puis, après lavage, à mettre en contact lesdites protéines avec les échantillons de sérums des patients testés susceptibles de contenir des auto-anticorps anti-Csk afin de réaliser une réaction immunologique entre les protéines Csk et les auto-anticorps Csk, et à révéler les complexes immunologiques éventuellement formés par tout moyen approprié pour déterminer la présence ou l'absence d'auto-anticorps anti-Csk dans les échantillons testés.
De manière classique, un moyen approprié pour révéler les complexes immunologiques éventuellement formés consiste à utiliser un anticorps anti-IgG humain couplé à la peroxydase, lequel est capable de se fixer sur les complexes éventuellement formés, puis après addition du substrat de la peroxydase de mesurer la réaction colorée par mesure de l'absorbance à 405 nm dont l'intensité est proportionnelle à la quantité d'anticorps présents.

On entend par forme modifiée ou fragment(s) de la protéine Csk, toutes protéines et tous polypeptides ou peptides dont la séquence en acides aminés est issue de celle de la protéine Csk, dès lors qu'ils conservent la capacité de former des complexes immunologiques spécifiques avec les auto-anticorps anti-Csk. On entend par conjugué de la protéine Csk, d'une forme modifié ou fragment(s) de celle-ci, les produits de couplage avec une molécule ou une substance porteuse.

L'invention se rapporte aussi à l'utilisation de la protéine Csk ou une forme modifiée, fragment(s) ou conjugués de ceux-ci pour la recherche d'auto-anticorps anti-Csk dans un échantillon biologique d'un sujet.
La protéine Csk, une forme modifiée ou fragments(s) de celle-ci peuvent être obtenus par purification à partir de tissus suivi éventuellement de clivage ou traitement visant à modifier le séquence, ou encore être produits par génie génétique en utilisant un système d'expression approprié comme un système baculovirus/cellules d'insecte (M. Koegl et al., *Biochem. J.,* 1994, 302, 737-744).

Le procédé selon l'invention offre l'avantage de pouvoir être réalisé sur le sérum des sujets ce qui en facilite grandement la mise en oeuvre par rapport à l'analyse de biopsies. En outre, comme cela est rapporté dans la partie expérimentale ci-après, il permet un diagnostic très précoce de la présence de tumeur, plus précoce qu'un diagnostic avec les marqueurs classiques comme ACE, CA153 ou même la protéine P53. Le procédé de l'invention permet le diagnostic précoce de cancers dans lesquels la protéine Src est activée : cancer du colon (Cartwright et al., *Proc. Natl. Acad. Sci.* USA, 1990, 87:558-562), mélanome (Loganzo et al., *Oncogene*, 1993, 8:2637-2644), cancer du sein (Lutrell et al., *Proc. Natl. Acad. Sci.* USA, 1994, 91:83-87; Chedin et al., *Carcinogenesis,* 1997, 18:1463-1472), cancer du colon et de l'ovaire (Budde et al., *Cancer Biochemistry and Biophysics,* 1994, 14:171-175), cancer de la vessie (Bénistant et al., en préparation). Il s'agit principalement des cancers de l'épithélium, donc tous les carcinomes qui sont les cancers les plus fréquents, certains mélanomes, certains cancers du sein. En outre, le procédé de l'invention qui permet un dépistage précoce du cancer devrait permettre de détecter des patients porteurs de polypes coliques en cours de transformation tumorale. A l'inverse, il est possible que le procédé de l'invention ne permette pas de détecter des cancers dans lesquels la protéine Src n'est pas activée, comme par exemple des cancers du poumon à petites cellules (Budde et al., *Cancer Biochemistry and Biophysics,* 1994, 14:171-175). Ceci confère au procédé de l'invention une certaine spécificité.

L'invention concerne également une trousse de diagnostic pour la mise en oeuvre d'un procédé de diagnostic précoce de cancers dans lesquels la protéine Src est activée comprenant :
- la protéine Csk ou une forme modifiée, fragment(s) ou conjugués de ceux-ci;
- des réactifs pour la constitution d'un milieu propice à la réaction immunologique entre les protéines Csk ou un forme modifiée, fragment(s) ou conjugués de ceux-ci et les auto-anticorps anti-Csk éventuellement présents dans un échantillon biologique;
- un ou plusieurs réactifs éventuellement marqués aptes à réagir avec les protéines Csk ou une forme modifiée, fragment(s) ou conjugués de ceux-ci et/ou les auto-anticorps anti-Csk et/ou les complexes immunologiques, pour la détection desdits complexes immunologiques éventuellement formés;
- le cas échéant, une protéine de référence ou un milieu biologique de référence.
   La protéine de référence est par exemple de la BSA, et le milieu biologique de référence est par exemple des sérums de patients présentant trois niveaux de réactivité : faible, moyen, fort.

D'autres avantages et caractéristiques de l'invention apparaîtront dans la description des travaux expérimentaux ayant conduit à la présente invention.

### I - Mise en évidence de l'existence des auto-anticorps anti-Csk et de leur intérêt comme marqueur précoce d'un cancer.

### 1) Mise en évidence et spécificité des auto-anticorps anti-Csk.

Les protéines antigènes Csk et deux membres de la famille Src (Src et FYN), ainsi que la protéine de contrôle BSA, ont été placés dans les puits des plaques ELISA à la même concentration de 10 µg/ml. Puis ont été ajoutés, les sera dilués au 1/20ème de deux patients sains (témoins), de quatre patients souffrant de cancer du colon à différents stades anatomopathologiques et d'un patient souffrant de cancer de la vessie de stade précoce. Les analyses ont été réalisées en duplicate. Les résultats représentant les valeurs des absorbances à 405 nm mesurées dans une expérience type sont rapportés dans le tableau I ci-dessous.

**Tableau I**

| Individus testés | Antigène Src | Antigène FYN | Antigène BSA | Antigène Csk |
|---|---|---|---|---|
| Témoin | 0,275 | 0,274 | 0,242 | 0,336 |
| Témoin | 0,289 | 0,279 | 0,263 | 0,281 |
| Cancer colon No. 1 stade C | 0,349 | 0,259 | 0,262 | 0,321 |
| Cancer colon No. 2 stade B | 0,276 | 0,267 | 0,250 | 0,789 |
| Cancer colon No. 3 stade D | 0,345 | 0,248 | 0,273 | 0,290 |
| Cancer colon No. 4 stade B | 0,310 | 0,280 | 0,271 | 0,375 |
| Cancer vessie No. 5 stade PTa | 0,322 | 0,283 | 0,215 | 1,360 |

On constate que les témoins, ainsi que les patients No. 1, 3 et 4 donnent une réponse faible quelque soit l'antigène. Par contre, les patients No. 2 et No. 5 donnent une réponse forte vis-à-vis de l'antigène Csk. Cette réponse est spécifique puisque celle obtenue avec les autres antigènes est faible. Les valeurs mesurées pour les antigènes Src, FYN et BSA sont globalement similaires. Ainsi pour simplifier, la suite des tests a été réalisée en utilisant uniquement l'antigène BSA (commercial) comme contrôle négatif. Les deux patients considérés comme positifs dans ce premier test sont atteints de cancer de stade relativement précoce : stade B du cancer du colon (classification Dukes) et stade PTa de cancer de la vessie (classification internationale). Enfin, ces deux patients appartiennent aux deux sexes : le patient No. 2 est une femme et le patient No. 5 est un homme.
Il convient de remarquer que les antigènes Src, FYN et Csk ont, tous les trois, été produits et purifiés selon un même protocole. La réponse antigénique étant uniquement observée par rapport à Csk, il est évident que les résultats obtenus ne peuvent être dus à la différence de mode de préparation ou à l'origine des protéines antigéniques. Par ailleurs, la spécificité de la liaison anticorps circulants / antigène Csk a été testée sur des sérums de patients positifs en réalisant une étude de déplacement *in vitro.* Dans cette expérience, le sérum des patients positifs a été mis en contact avec les antigènes Csk et BSA, puis Csk ou BSA en solution (10 ou 100 µg/ml) ont été rajoutés. En présence de Csk (10 µg/ml), on observe une diminution de l'absorbance. Avec 100 µg/ml de la protéine Csk, l'absorbance revient à la valeur de base. Dans les mêmes conditions, la protéine BSA est sans effet. Ceci permet de réaffirmer que les auto-anticorps anti-Csk sont spécifiques de l'antigène Csk.

### 2) Les auto-anticorps anti-Csk permettent de détecter spécifiguement le cancer.

La présence des auto-anticorps anti-Csk a été recherchée chez 92 patients atteints de diverses formes de cancer, 30 cancers colorectaux, 17 cancers de la vessie, 7 cancers du poumon à petites cellules, 21 cancers du sein, 10 cancers de l'ovaire, ainsi que chez 40 témoins donneurs du CTS considérés comme sains et chez 21 patients souffrant d'autres maladies (11 patients souffrant de maladies auto-immunes de la tyroïde et 10 patients atteints de pathologies gastrointestinales non cancéreuses diverses). Les tests ELISA ont été réalisés vis-à-vis de la protéine Csk, en utilisant en parallèle BSA comme contrôle négatif. Toutes les mesures ont été réalisées en duplicate ou quadruplicate. Le sérum du patient No. 5 pour lequel la réponse était la plus forte dans le tableau I a été inclus systématiquement dans chacune des plaques de dosage comme contrôle positif, l'absorbance de ce sérum vis-à-vis de Csk dépassant toujours la valeur de 1,2. Il convient de remarquer que l'absorbance de ce sérum dans le test ELISA est restée constante pendant un longue période ce qui démontre la stabilité des auto-anticorps anti-Csk. Les valeurs obtenues pour chaque sérum et pour chacune des conditions expérimentales ont été moyennées. Les valeurs d'absorbance vis-à-vis de BSA ont été inférieures à 0,4 dans 90% des cas. Ont été considérés comme positifs les individus dont l'absorbance vis-à-vis de Csk est inférieur à 0,4 et dont le rapport des absorbances Csk/BSA est supérieur à 1,3, comme cela avait été décrit pour la recherche des auto-anticorps anti-P53 (C. P. Wild et al., 1995, *Int. J. Cancer (Pred. Oncol.)*: 64, 176-181). Les résultats obtenus sont rapportés dans le tableau 2 ci-dessous.

**Tableau 2**

| Population | Nombre de patients positif / Nombre testé | % |
|---|---|---|
| Cancer colorectal | 6 / 30 | 20 |
| Cancer de la vessie | 3 / 17 | 18 |
| Adénocarcinome pulmonaire | 1 / 7 | 14 |
| Cancer du poumon à petites cellules | 0 / 7 | 0 |
| Cancer du sein | 1 / 21 | 5 |
| Cancer de l'ovaire | 1 / 10 | 10 |
| Témoins sains | 0 / 40 | 0 |
| Autres maladies | 0 / 21 | 0 |

Les résultats présentés dans le tableau 2 montrent que le test est spécifique de la pathologie cancéreuse. En effet, aucun des témoins ne donne une réponse positive, ni aucun des patients souffrant de pathologies non cancéreuses tels que ceux souffrant de maladies auto-immune de la thyroïde qui présentaient une forte charge en auto-anticorps anti-thyroïde. Par contre, des patients ont été détectés dans tous les types de cancer testés, à l'exception de ceux atteints d'un cancer du poumon à petites cellules. Ce dernier résultat pourrait être lié au faible échantillonnage ou au fait que la protéine Csk n'intervient pas dans ce type de tumeur.

### 3) Le taux des auto-anticorps anti-Csk diminue après résection de la tumeur.

Certains des patients atteints d'un cancer du colon qui ont été testés dans cette étude étaient en parallèle dans un autre protocole d'analyse P53 et lés sérums avaient été prélevés en pré et post opératoire. Parmi les six patients positifs en auto-anticorps anti-Csk rapportés dans le tableau 2, trois ont pu ainsi être suivis. Les résultats obtenus sont présentés dans le tableau 3 ci-dessous qui donnent les valeurs de DO obtenues vis-à-vis de l'antigène Csk. Ces résultats montrent une diminution du taux d'auto-anticorps anti-Csk dans le sérum des patients après résection de la tumeur.

**Tableau 3**

| Patients | Pré-opératoire | Post-opératoire |
|---|---|---|
| 1 | 0,583 | 0,496 |
| 2 | 0,526 | 0,318 |
| 3 | 1,256 | 0,444 |

Ces résultats permettent de réaffirmer que les auto-anticorps anti-Csk sont spécifiques du cancer, car les patients considérés ont été opérés pour cette pathologie. Ces résultats montrent l'intérêt que présente en outre le dosage des auto-anticorps anti-Csk dans le suivi des patients.

### 4) Les auto-anticorps anti-Csk sont présents chez des patients atteints de cancers des stades précoces.

La répartition des patients positifs versus le nombre de patients testés en fonction du stade d'évolution de la tumeur et pour les cas de cancers colorectaux et de la vessie est rapportée dans le tableau 4 ci-dessous.

**Tableau 4**

| Stade | Précoce | Tardif |
|---|---|---|
| colorectal | 4 / 15 | 2 / 15 |
| vessie | 3 / 17 | 0 / 17 |

La plus grande fréquence de détection est observée pour les stades tumoraux précoces : stades A et B du cancer du colon selon la classification de Dukes, et stades PTa et PT1 du cancer de la vessie selon la classification internationale.

### 5) Nature des immunoglobulines mises en jeu.

Afin d'identifier la nature des immunoglobulines mises en jeu, la réactivité des classes d'immunoglobulines suivantes : IgGFc, IgG1, IgG2, IgG3, IgG4, IgGA et IgGM couplées à la peroxidase ont été testées chez 3 patients positifs. Le tableau 5 ci-dessous rapporte les valeurs de DO obtenues vis-à-vis de l'antigène Csk.

**Tableau 5**

| Type d'IgG | IgGFc | IgG1 | IgG2 | IgG3 | IgG4 | IgGM | IgGA |
|---|---|---|---|---|---|---|---|
| Patient 1 | 0,453 | 0,288 | 0,221 | 0,172 | 0,144 | 0,551 | 0,205 |
| Patient 2 | 0,459 | 0,189 | 0,155 | 0,137 | 0,134 | 0,502 | 0,170 |
| Patient 3 | 1,962 | 1,313 | 0,245 | 0,123 | 0,082 | 0,585 | 0,410 |

La classe majoritaire chez 2 des 3 patients est la classe IgGM, ce qui renforce le fait que les auto-anticorps anti-Csk sont issus d'un phénomène immunitaire précoce. Chez le patient No. 3 qui donne une réponse très forte, on observe une amplification de la réponse immune et une génération d'anticorps de type IgG1.

### 6) Les auto-anticorps anti-Csk sont observés chez des patients qui présentent effectivement une élévation du taux de leur protéine Csk dans les tissus tumoraux comparativement au tissu sain.

L'étude a été menée sur des échantillons de tissu sain, prélevés à distance de la tumeur, et sur des échantillons prélevés directement sur la tumeur des patients. Le contenu en protéine endogène Csk dans ces tissus a été analysé par la technique de Western Blotting.

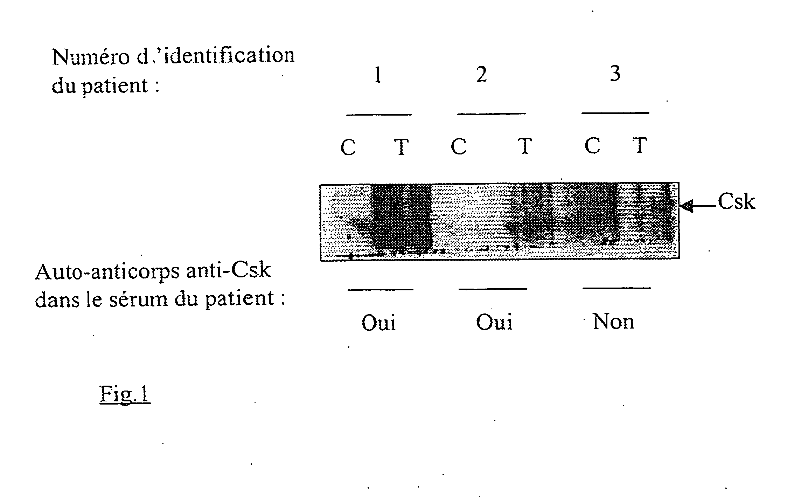

La figure 1 en annexe montre le contenu en protéine endogène Csk dans le tissu sain (C) et tumoral (T) de 3 patients : en (1) vessie, en (2) et (3) colon. On observe que le contenu en protéine endogène Csk du patient No. 5 du tableau 1 et de l'un des patients positifs atteints d'un cancer du colon du tableau 2 augmente considérablement dans le tissu tumoral (T) comparativement au tissu sain (C); au contraire, le contenu en Csk d'un patient négatif en auto-anticorps anti-Csk est inchangé.

### II - Pertinence du dosage des auto-anticorps anti-Csk.

Les 30 patients atteints d'un cancer du colon étudiés dans le cadre de la présente invention faisaient également partie d'un protocole d'étude portant sur la recherche des auto-anticorps P53.
Les marqueurs tumoraux classiques ACE et le CA 19.9 de ces patients ont également été déterminés. Les données des différents tests sont regroupés dans le tableau 6 ci- dessous.

**Tableau 6**

| Patients | Stade | ACE | CA 19.9 | P 53 | Csk |
|---|---|---|---|---|---|
| | Précoce | | | | |
| 1 | A | - | - | + | + |
| 2 | A | - | - | - | - |
| 3 | B | - | - | - | - |
| 4 | B | nd | nd | - | - |
| 5 | B | - | - | - | - |
| 6 | B | - | - | - | + |
| 7 | B | - | - | - | - |
| 8 | B | - | - | - | - |
| 9 | B | - | + | - | + |
| 10 | B | + | + | - | - |
| 11 | B | - | - | - | - |
| 12 | B | + | + | - | - |
| 13 | B | - | - | - | - |
| 14 | B | nd | nd | - | - |
| 15 | C | - | - | + | + |

| | Tardif | | | | |
|---|---|---|---|---|---|
| 16 | C | - | - | - | - |
| 17 | C | - | - | + | - |
| 18 | C | - | + | - | - |
| 19 | C | - | - | - | + |
| 20 | C | + | - | - | - |
| 21 | C | nd | nd | - | - |
| 22 | C | nd | nd | - | - |
| 23 | C | + | + | - | - |
| 24 | C | - | - | - | + |
| 25 | C | + | + | - | - |
| 26 | D | - | + | + | - |
| 27 | D | + | + | - | - |
| 28 | D | - | - | + | - |
| 29 | D | + | + | + | - |
| 30 | D | - | - | + | - |
| (nd : non déterminé) | | | | | |

Le tableau 6 montre qu'aucun des marqueurs ne permet un dépistage absolu du cancer colorectal: Cependant, ce tableau montre que le test Csk permet d'une part, de détecter des patients atteints d'un cancer qui ne sont détectés par aucun des autres tests (patients No. 6, 19 et 24) et d'autre part, de diagnostiquer plus de cancers à un stade précoce.
Il faut remarquer que les patients testés atteints par d'autres types de cancer, tels que les cancers du poumon, de l'ovaire ou du sein illustrés dans le tableau 2, ont été choisis car ils portent tous les marqueurs respectifs de leur cancer (CYFRA 21, CA 125, CA15.3). Ces patients développent donc un cancer de manière certaine. Cependant, le dosage des auto-anticorps Csk étant un dépistage de la maladie à un stade précoce, il est possible d'expliquer le faible nombre de patients positifs détectés par le fait que ces patients sont à un stade trop avancé de leur cancer.

## Revendications

1. Procédé de diagnostic précoce d'un cancer dans lequel la protéine Src est activée chez un patient consistant à identifier par toute méthode appropriée la présence d'auto-anticorps dirigés contre la protéine Csk dans un échantillon biologique prélevé chez ledit patient.

2. Procédé selon la revendication 1, **caractérisé en ce que** les auto-anticorps dirigés contre la protéine Csk sont identifiés par une réaction immunologique entre lesdits auto-anticorps et les protéines Csk, une forme modifiée, fragment(s) ou conjugués de ceux-ci.

3. Procédé selon la revendication 2, **caractérisé en ce** les auto-anticorps dirigés contre la protéine Csk sont identifiés par la technique ELISA.

4. Utilisation de la protéine Csk ou une forme modifiée, fragment(s) ou conjugués de ceux-ci pour la recherche d'auto-anticorps anti-Csk dans un échantillon biologique d'un sujet.

5. Trousse de diagnostic pour le diagnostic précoce d'un cancer dans lequel la protéine Src est activée dans un échantillon biologique d'un sujet comprenant :
- la protéine Csk ou une forme modifiée, fragment(s) ou conjugués de ceux-ci;
- des réactifs pour la constitution d'un milieu propice à la réaction immunologique entre les protéines Csk ou une forme modifiée, fragment(s) ou conjugués de ceux-ci et les auto-anticorps anti-Csk éventuellement présents dans un échantillon biologique;
- un ou plusieurs réactifs éventuellement marqués aptes à réagir avec les protéines Csk ou une forme modifiée, fragment(s) ou conjugués de ceux-ci et/ou les auto-anticorps anti-Csk et/ou les complexes immunologiques, pour la détection desdits complexes immunologiques éventuellement formés;
- le cas échéant, une protéine de référence ou un milieu biologique de référence.

## Patentansprüche

1. Verfahren zur frühen Diagnose eines Krebses, in dem das Src Protein bei einem Patienten aktiviert ist, das darin besteht, durch jedwede angepasste Methode die Anwesenheit von Auto-Antikörpern zu identifizieren, die gegen das Csk Protein in einer biologischen Probe, die bei dem Patienten entnommen wurde, gerichtet sind.

2. Verfahren nach Anspruch 1, **dadurch gekennzeichnet, dass** die Auto-Antikörper, die gegen das Csk Protein gerichtet sind, durch eine immunologische Reaktion zwischen den Auto-Antikörpern und den Csk Proteinen, einer modifizierten Form, Fragment (en) oder daraus konjugierten identifiziert werden.

3. Verfahren nach Anspruch 2, **dadurch gekennzeichnet, dass** die Auto-Antikörper, die gegen das Csk Protein gerichtet sind, durch die ELISA Technik identifiziert werden.

4. Verwendung des Csk Proteins oder einer modifizierten Form, Fragment(e) oder daraus konjugierte zur Suche von Anti-Csk Auto-Antikörpern in einer biologischen Probe von einem Subjekt.

5. Diagnoseetui für die frühe Diagnose eines Krebses, in dem das Src Protein in einer biologischen Probe von einem Subjekt aktiviert ist, mit Folgendem:
- das Csk Protein oder eine modifizierte Form, Fragment(e) oder daraus konjugierte;
- Reagenzien für die Bildung eines Milieus, das vorteilhaft ist für die immunologische Reaktion zwischen den Csk Proteinen oder einer modifizierten Form, Fragment(en) oder daraus konjugierten und die Anti-Csk Auto-Antikörper, die ggf. in einer biologischen Probe anwesend sind;
- ein oder mehrere Reagenzien, die ggf. markiert sind, die fähig sind mit den Csk Proteinen oder einer modifizierten Form, Fragment(en) oder daraus konjugierten und/oder den Anti-Csk Auto-Antikörpern und/oder den immunologischen Komplexen zu reagieren, für die Erkennung der immunologischen Komplexe, die ggf. gebildet sind;
- ggf. ein Bezugsprotein oder ein biologisches Bezugsmilieu.

## Claims

1. Method for early diagnosis of a cancer in which the Src protein is activated in a patient, consisting of identifying by any appropriate method the presence of autoantibodies directed against the Csk protein in a biological sample taken from said patient.

2. Method according to claim 1, **characterised in that** the autoantibodies directed against the Csk protein are identified by an immunologic reaction between said autoantibodies and the Csk proteins, a modified form, fragment(s) or conjugates thereof.

3. Method according to claim 2, **characterised in that** the autoantibodies directed against the Csk protein are identified by the ELISA technique.

4. Use of the Csk protein or a modified form, fragment(s) or conjugates thereof for the search for anti-Csk autoantibodies in a biological sample of a subject.

5. Diagnostic kit for early diagnosis of a cancer wherein the Src protein is activated in a biological sample of a subject, comprising:
- the Csk protein or a modified form, fragment(s) or conjugates thereof;
- reagents for constitution of a favourable medium for the immunologic reaction between the Csk proteins or a modified form, fragment(s) or conjugates thereof and the anti-Csk autoantibodies possibly present in a biological sample;
- one or several possibly marked reagents able to react with the Csk proteins or a modified form, fragment(s) or conjugates thereof and/or the anti-Csk autoantibodies and/or the immunologic complexes, for detection of said possibly formed immunologic complexes;
- if applicable, a reference protein or a reference biological medium.
